# EUROPEAN PATENT SPECIFICATION

(11) **EP 1 928 857 B1**
(45) Date of publication and mention of the grant of the patent: **23.06.2010**
(21) Application number: 06793769.8
(22) Date of filing: 22.09.2006
(51) Int. Cl.: C07D 401/12, C07D 239/26, C07D 403/06

(54) **METHOD FOR PREPARING (3-CHLORO-4-FLUOROPHENYL)-(4-FLUORO-4-{[(5METHYL-PYRIMIDIN-2-YLMETHYL)-AMINO]-METHYL}-PIPERIDIN-1-YL)-METHANONE AND NOVEL INTERMEDIATE PYRIMIDINE DERIVATIVES**
VERFAHREN ZUR HERSTELLUNG VON (3-CHLOR-4-FLUORPHENYL)-(4-FLUOR-4-{[(5METHYLPYRIMIDIN-2-YLMETHYL)AMINO]METHYL}PIPERIDIN-1-YL)METHANON UND NEUE PYRIMIDINDERIVATZWISCHENPRODUKTE
PROCÉDÉ DE PRÉPARATION DE LA (3-CHLORO-4-FLUOROPHÉNYL)-(4-FLUORO-4-{[(5-MÉTHYL-PYRIMIDIN-2-YLMÉTHYL)-AMINO]-MÉTHYL}-PIPÉRIDIN-1-YL)-MÉTHANONE ET NOUVEAUX DÉRIVÉS PYRIMIDINE INTERMÉDIAIRES

(30) Priority: 27.09.2005 FR 0509852
(43) Date of publication of application: 11.06.2008
(73) Proprietor: PIERRE FABRE MEDICAMENT, 92100 Boulogne-Billancourt (FR)
(72) Inventor: VACHER, Bernard, F-81100 Castres (FR); MAUREL, Jean-Louis, F-81100 Burlats (FR); BRUNEL, Serge, F-81100 Castres (FR)
(74) Representative: Ahner, Francis
(86) International application number: PCT/EP2006/066658
(87) International publication number: WO 2007/039499

(56) References cited:
- WO-A-02/064585
- WO-A-03/106449
- ISODA S ET AL: "Medicinal chemical studies on antiplasmin drugs. VI. Aza analogs of 4-aminomethylbenzoic acid" CHEMICAL AND PHARMACEUTICAL BULLETIN, vol. 28, no. 5, May 1980 (1980-05), pages 1408-1414, XP002387618

## Description

The present invention relates to a method for preparing (3-chloro-4-fluorophenyl)-(4-fluoro-4-{[(5-methyl-pyrimidin-2-ylmethyl)-amino]-methyl}-piperidin-1-yl)-methanone, having formula (2)

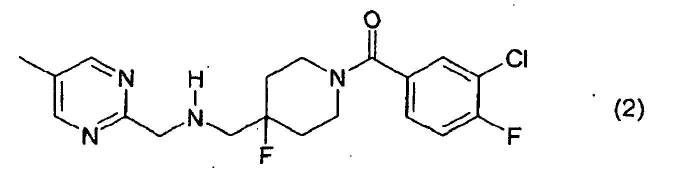

Compound (2), claimed in international application WO 03/106449, is an effective, selective agonist of serotonergic receptors of sub-type 5-HT_{1A}. In this respect, it is potentially useful for the treatment of pathologies associated with disorders of the serotonergic system. Owing to its major therapeutic potential, a method·for synthesizing the compound of formula (2) which can be given industrial applicability appears highly desirable.

International application WO 02/064585 describes a novel method for preparing structural analogs of compound (2). However, the application of said method for preparing molecule (2) assumes the prior determination of a method for preparing 5-methyl-pyrimidine-2-methylamine of formula (1). In addition, in unexplained manner, in the particular case of preparing the compound of formula (2), the application of said method does not give satisfactory results. On this account, the present invention also concerns a modification of the prior method (WO 02/064585) so as to make the synthesis of the formula (2) compound applicable on an industrial scale.

The present invention concerns a method for preparing (3-chloro-4-fluorophenyl)-(4-fluoro-4-{[(5-methyl-pyrimidin-2-ylmethyl)-amino]-methyl}piperidin-1-yl)-methanone of formula (2). 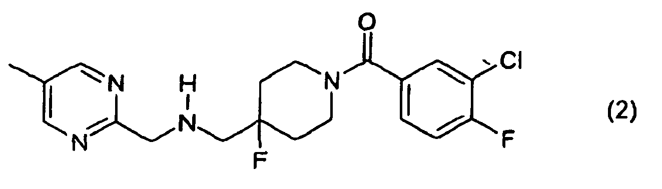 by condensation between 5-methyl-pyrimidin-2-methylamine of formula (1): 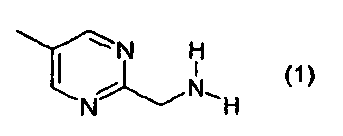 and cyanohydrin of formula (3):

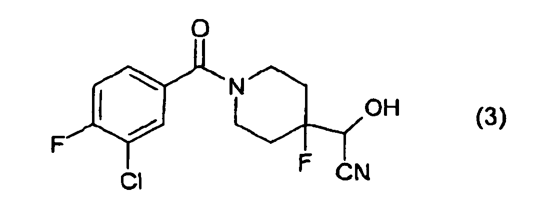

Said condensation being conducted in the presence of an agent able to trap the water released during the reaction. Advantageously said condensation is constituted in the presence of a molecular sieve.

As recalled previously, the preparation of the formula (2) compound according to the method described in WO 02/064585 is difficult to apply, in particular on account of its low yield (i.e. less than 30 %). The present invention has therefore improved on the prior art method to make it economically viable (i.e. yield greater than 50 %), more environmentally safe and hence globally more favourable for industrial application.

The chemistry of pyrimidines is well known (D.J.Brown, The Pyrimidines; Wiley & Sons: New York; 1962; M.G.Hoffman, A.Nowak, M.Müller, Pyrimidines in Methods of Organic Chemistry; Georg Thieme Verlag: Stuttgart, 1998). Therefore, the preparation method which consists of condensing a derivative of amidine type with a di-electrophil-1,3 belongs to conventional methods. To situate the prior art, the pyrimidine preparations reported in the following publications may be cited as examples: P.C.Mitter, J.C.Bardhan, Journal of the Chemical Society 1923, 123, 2179-84; C.C.Price, J.Zomlefer, J.Org.Chem.1949,14,210-215; A.Dornow, E.Neuse, Arch.Pharm.1954, 287, 361-376; H.Bredereck, H.Herlinger, E.H.Schweizer, Chem.Ber,1960,93,1208-1211; A.Tanaka, Y.Motoyama, H.Takasugi, Chem.Pharm.Bull.1994, 42, 1828-1834; WO 97/38995. However, surprisingly, the synthesis of 5-methylpyrimidine-2-methylamine has not been described in the chemical literature.

The advantage of the inventive method for obtaining 5-methyl-pyrimidine-2-methylamine lies in the use of a derivative of amidine type derived from glycine. The synthesis of compound (1) according to the invention therefore only comprises two steps and proceeds with a high yield. Starting from compound (1), the preparation of the formula (2)compound may be conducted following a method similar to the one described in WO 02/064585. However, it is particularly advantageous to modify said method according to the conditions specified in the present application.

According to a particular embodiment of the invention, 5-methyl-pyrimidine-2-methylamine of formula (1) is obtained from a pyrimidine derivative of formula (4): 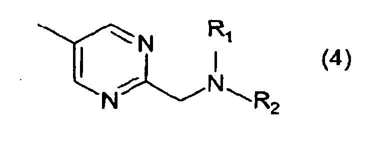 in which the radicals R₁ and R₂ separately represent a hydrogen atom and a *tert*-butyloxycarbonyl group or a benzyloxycarbonyl group; where R₁ and R₂ with their carrier nitrogen atom together form a phtalimido group.

The synthesis method for 5-methylpyrimidine-2-methylamine of formula (1) comprises two steps (schematic 1):

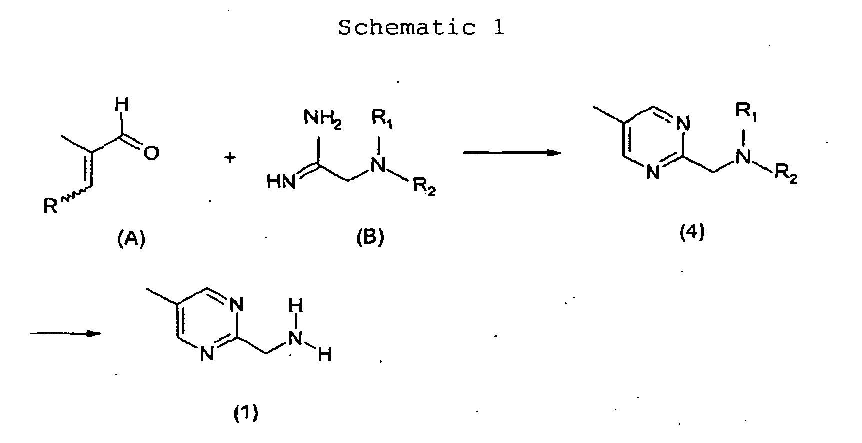

According to one advantageous variant of the present invention, the condensing of an amidine derived from glycine of formula (B) is conducted with a dipolarophil-1,3 of formula (A1) in which R₃ represents a releasable group such as a C₁ to C₄ alcoxy group for example, methoxy in particular, the double bond in compound (A1) possibly being of E or Z stereochemistry indifferently. 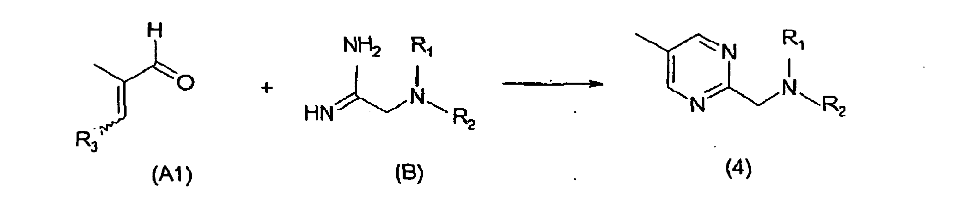 to give the compound of formula (4) in which the radicals R₁ and R₂ have the same meaning as previously.

The first step consists of causing to react a di-electrophil-1,3 of formula (A) with the suitable amidine of formula (B) to give a pyrimidine of formula (4) whose primary amine function is protected.

As examples of formula (A) compounds, the inventors used commercially available compounds such as 3-ethoxy-methacrolein (R is then an ethoxy group) [42588-57-8], 3-amino-2-methylacrolein (in this case R represents an amino group) [30989-81-2] and 2-methyl-3-dimethylaminoacrolein (in this case R is a dimethylamine group) [19125-76-9].

Similarly, various protective groups may be used to mask the primary amine function, for example at the formula (B) amidine, T.W.Greene, P.G.M.Wuts, Protective Groups in Organic Synthesis; Wiley & Sons: New York, 1999.

To illustrate the present invention, as glycine-derived amidines, the inventors used: 2-(N-tert-butoxycarbonylamino)-glycinamidine [251294-65-2], 2-(N-benzyloxycarbonylamino)glycinamidine [77390-81-9] and N-(methylamidine)-phtamidide. This latter compound [N-(methylamidine)-phtamidide] is not reported in the literature, but may easily be obtained by adding ammonia to the corresponding N-(cyanomethyl)-phtalimide [3842-20-4], following a method similar to the one described in Tetrahedron Letters 1999, 40, 7067-7071. The condensation between reagents (A) and (B) is then conducted in a basic medium under conditions identical to those indicated in the literature: J.Org.Chem, 1993, 58, 241-244 or Chem. Heterocycl. Compd. 1997, 33,843-846.

During the second step, the primary amine function is released by cleavage of the protective group according to conventional techniques well known to persons skilled in the art (e.g. T.W.Greene, P.G.M.Wuts, Protective Groups in Organic Synthesis; Wiley & Sons; New York, 1999). The 5-methylpyrimidine-2-methylamine (1) so formed can be purified, if so desired, by distillation or chromatography on silica gel and then salified if so wished. The method for preparing 5-methylpyrimdine-2-methylamine such as just described is sturdy and can be performed on semi-or fully industrial scale.

According to another variant of embodiment of the invention, the 5-methylpyrimidine-2-methylamine of formula (1) is obtained from a pyrimidine derivative of formula (4-1) 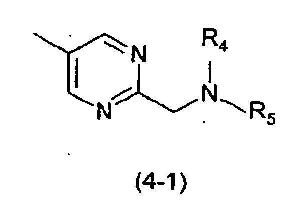 itself obtained by condensing a dipolarophil-1,3 of formula (A) or formula (A1), (A) and (A1) having the same meanings as given previously, with a glycine-derived amidine of formula (B1) in which the radicals R₄ and R₅ together or independently represent a hydrogen atom or a protective group, in particular an alkylcarbonyl group such as a trifluorocarbonyl group

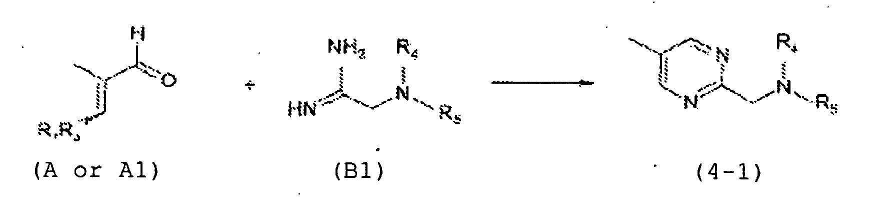

As mentioned above, one essential aspect of the present invention consists of an improvement on the method for synthesizing the compound of formula (2) using intermediate compounds (1) and (3).

Therefore, according to patent application WO 02/064585, the synthesis of compound (1) uses the condensing between an arylmethylamine and a cyanohydrin, typically in an alcohol medium, in the presence of a base, of a hydride-donor agent and an agent able to trap the cyanide ions. However, the application of these experimental conditions, for preparing compound (2) from intermediates (1) and (3), gives a yield of product (2) which is unsatisfactory (i.e. less than 30 %). The inventors have discovered that the use of a molecular sieve during the reaction between (1) and (3) makes it possible to improve the yield of product (2) quite significantly (i.e. yield of more than 50 %). This improvement on the method reduces the quantity of effluent, facilitates purification of the active ingredient (2) and considerably reduces operating costs.

Finally, the present invention also extends to novel chemical products evidenced throughout the above-described methods, and in particular the following products:
- 5-methyl-pyrimidine-2-methylamine of formula (1) 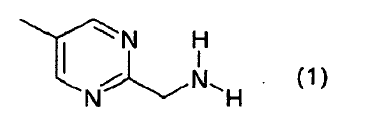
- pyrimidine derivatives of formula (4) 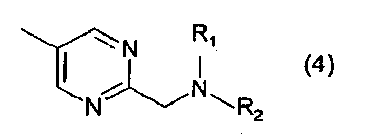 in which the radicals R₁ and R₂ independently represent a hydrogen atom and a *tert*-butyloxycarbonyl group or a benzyloxycarbonyl group; where R₁ and R₂ with their nitrogen atom carrier together form a phtalimido group.
- pyrimidine derivatives of formulas (4a),(4b) and (4c)

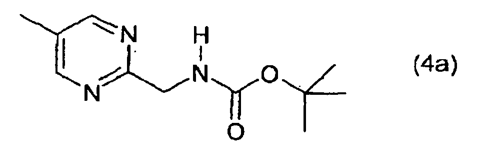

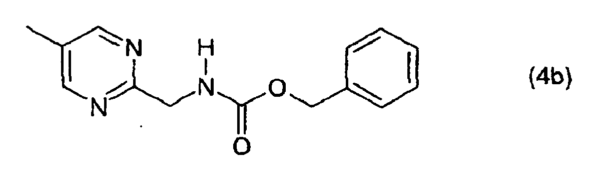

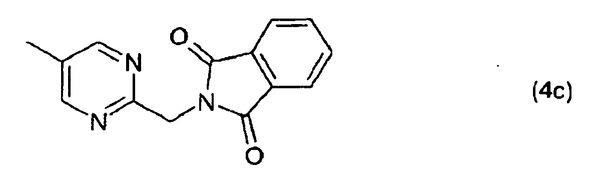

The following examples illustrate the invention.

### Example 1: 5-methyl-2-(N-tert-butoxycarbonyl-amino) pyrimidine (4a)

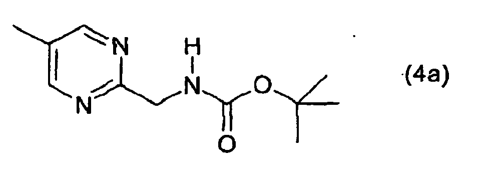

### Method A: using 3-ethoxymethacrolein

32 g sodium (1.39 gram atom) are added to 2.5 L methanol, the temperature being controlled by means of a cold water bath. When the sodium is fully dissolved, 70g of 3-ethoxymethacrolein are added (0.613 mol) then 69.3 g (0.4 mol) of *tert*-butoxycarbonylaminomethyl-amidine (prepared according to: Tetrahedron Lett. 1999, 40, 7067-7071). The mixture is placed under solvent reflux for 5h then evaporated. The residue is collected in water then extracted with dichloromethane. The organic phases are dried on magnesium sulphate then evaporated. The residue is crystallized in cold hexane, and the product is recovered by filtration in the form of a white solid, 67.4 g (75 %).

### Method B: using 3-amino-2-methylacrolein

A suspension of 0.5 g of 3-amino-2-methylacrolein (5.9 mmol), 1.89 g of N-(*tert*-butyloxycarbonyl)-2-aminoacetonitrile (12.2 mmol), 1.63 g of K₂CO₃ (11.8 mmol) in 50 mL ethanol is refluxed for 24 h. The insolubles are filtered , the ethanol evaporated and the residue purified on silica gel using as eluent a mixture of dichloromethane 95/methanol 4.5/ammonia 0.5. A white solid weighing 1.04 g is obtained (79 %).

### Method C: using 2-methyl-3-dimethylaminoacrolein

A solution of 1 g (5.8 mmol) *tert*-butoxycarbonylaminomethyl-amidine (prepared according to Tetrahedron Lett. 1999, 40, 7067-7071), 0.65 g sodium methylate (11.8 mmol.) and 0.79 g of 2-methyl-3-dimethylaminoacrolein (6.9 mmol) in 10 mL ethanol is refluxed for 5 h then evaporated. The residue is collected in water then extracted with dichloromethane. The organic phases are washed in water, salified and dried on magnesium sulphate, then concentrated. The residue is purified on silica gel using as eluent a mixture of dichloromethane 97/methanol 3. In this way 110 mg of product are recovered (4a).
Analyses of compound (4a):
melting point: 92°C
¹H-NMR (DMSOd₆); δ 8.59 (s,2H); 7.44 (t,1H); 4.26 (d,2H); 2.25 (s, 3H); 1,38 (s, 9H).

### Example 2: 5-methyl-2-(N-benzyloxycarbonylamino) pyrimidine (4b).

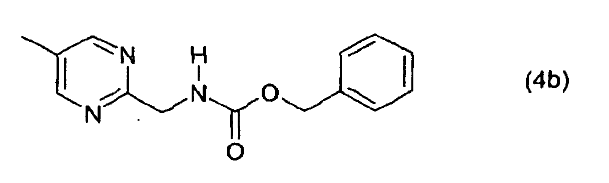

5.01 g N-(benzyloxycarbonyl)-2-aminoacetonitrile (26.3 mmol) and 0.49 g of N-acetylcystein (3 mmol) are dissolved in 30 mL methanol. 2.39 g ammonium acetate (31 mmol) are added and left under agitation 48 h at 50°C. The methanol is evaporated under reduced pressure and the residue collected in a water/dichloromethane mixture, decanted and the aqueous phase is evaporated under reduced pressure. The white solid obtained is vacuum dried (5.45g of benzyloxycarbonylaminomethylamidine).

2.68 g sodium (0.116 gram atom) are added in portions to 200 mL methanol controlling the temperature by means of a cold water bath. When the sodium is fully dissolved, 3 g of 3-ethoxymethacrolein (26.3 mmol)are added then 5.45 g benzylcarbonylaminomethylamidine (26.3 mmol) prepared previously. The mixture is heated in a reflux for 5 h, then the methanol is evaporated under reduced pressure. The residue is collected in water and the aqueous phase is extracted with dichloromethane, the organic phases are dried on magnesium sulphate' and concentrated. The product is purified on silica gel using as eluent a mixture of dichloromethane 95/methanol 4.5/ ammonia 0.5. 2.25 g of the titer product are obtained in the form of a white solid (33 %).
Analysis of compound (4b)
MS: ESI⁺; MH⁺ = 258.10.

### Example 3: N-[(5-methyl)-pyrimidin-2-ylmethyl)]-phtalimide (4c)

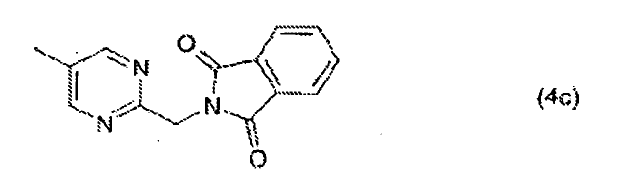

0.48 g of sodium hydride (12 mmol) are placed in suspension in 20 mL tetrahydrofurane, then 0.5 g of 3-amino-2-methylacrolein (5.9 mmol) are added and the mixture left under agitation for 40 min at room temperature. 1.1 g of phtalimidoacetonitrile (5.9 mmol) are added and heated under reflux for 17 h. The insolubles are filtered and the filtrate concentrated under reduced pressure. The titer compound is recovered in the form of a yellow solid (0.3 g).
Analyses of compound (4c):
MS: APCI⁺; MH⁺ = 254.1
¹H-NMR (DMSOd₆): δ 8.58 (s, 2H); 7.91 (m, 4H); 4.96 (s,2H); 2.23 (s,3H).

### Example 4: 5-methylpyrimidine-2-methylamine (1)

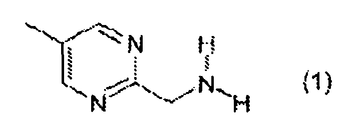

### Method A: using 5-methyl-2-(N-tert-butoxycarbonylamino)pyrimidine (4a).

67 g of 5-methyl-2-(N-*tert*-butoxycarbonylamino) pyrimidine (4a) (0.3 mol) are left under agitation 4 h at room temperature in 1 L of a 5 N HCl solution in isopropanol. When dissolved, the hydrochlorate of the titer compound precipitates. The precipitate is filtered and washed in isopropanol and then in heptane, and is vacuum dried. 57.7 g (98 %) of compound (1) is obtained in dihydrochloride form.

### Method B: using 5-methyl-2-(N-benzyloxycarbonylamino) pyrimidine (4b).

Hydrogenolysis of the formula (4b) compound is conducted following the method reported in: Chem. Pharm. Bull. 1980, 28(5), 1408-1414.

### Method C: using N-[(5-methyl)-pyrimidin-2-ylmathyl)]-phtalimide (4c).

A solution of 0.3 g (1.2 mmol) of N-[(5-methyl)-pyrimidin-2-ylmethyl)]-phtalimide (4c) in 20 mL ethanolamine is left under agitation 3 h at 70°C. The solution is diluted with water then extracted with dichloromethane. The combined organic phases are dried on magnesium sulphate and concentrated. The residue is dissolved in isopropanol and crystallized through the addition of a 5N HCl solution in isopropnaol. The precipitate is filtered then washed in isopropanol and heptane. It is then vacuum dried to yield compound (1) in the form of a dihydrochloride (0.18 G, 78 %). Analyses of the dihydrochloride compound (1):
Melting point: decomposition on and after 240°C;
C₆H₁₁N₃C₁₂: 196.08
% calculated: C 36.75; H 5.56; N 21.43
% found: C 36.52; H 5.35; N 21.27
¹H-NMR (DMSOd₆): δ 11.67 (s,1H); 8.73 (s, 2H); 8.67 (s, 3H); 4.2 (m, 2H); 2.31 (s, 3H).

### Example 5: (3-chloro-4-fluorophenyl)-(4-fluoro-4-{[(5-methylpyrimidin-2-ylmethyl)-amino]-methyl}-piperidin-1-yl)-methanone (2).

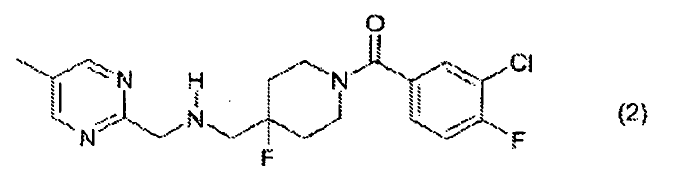

A mixture of 8.31 g (42.4 mmol) 5-methylpyrimidin-2-methylamine dihydrochloride (1), 13.29 g (42.2 mmol) cyanohydrin(3), 14.7 g of 1,4-diazabicyclo[2,2,2]octane (131 mmol), 2.70 g of sodium cyanoborohydride (43 mmol), 13 g of 4Å molecular sieve and 250 mL methanol are left under agitation 6h at 50°C. The insolubles are filtered, the filtrate is concentrated under reduced pressure, the residue collected in water and extracted with dichloromethane. The combined organic phases are extracted using a 2 N HCL solution, the combined aqueous phases are washed in ethyl acetate. The aqueous phase is made basic through the addition of ammonia then extracted with dichloromethane, dried on magnesium sulphate and vacuum concentrated to yield the titer compound in the form of an oil (11.87 g, 71 %).
Analyses of the fumarate of compound (2)
Melting point: 105 °C (decomposition)
C₂₃H₂₅CIF₂N₄O₅: 510.93
% calculated: C 53'.39; H 4.85; N 10.38
% found: C 53.20; H 5.11; N 10.52
¹H-NMR (DMSOd₆) : δ 1.67-1.91 (m, 4H); 2.26 (s, 3H); 2.60 (d, 2H); 3.10-3.40 (m, 3H); 3.92 (s; 2H); 4.24 (s, 1H); 6.61 (s, 2H); 7.42-7.61 (m, 2H); 7.66 (d, 1H); 8.62 (s, 2H).

## Claims

1. Method for preparing (3-chloro-4-fluoro-phenyl)-(4-fluoro-4-{[(5-methyl-pyrimidin-2-ylmethyl)-amino]-methyl}-piperidin-1-yl)-methanone of formula (2) 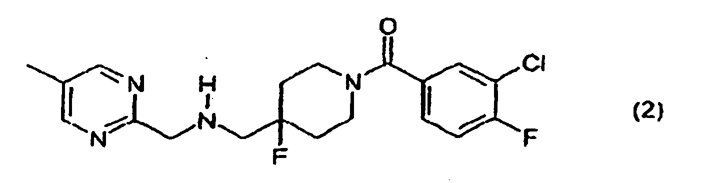 by condensation between 5-methyl-pyrimidin-2-methylamine of formula (1) 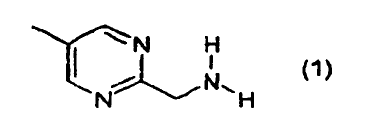 and cyanohydrin of formula (3) 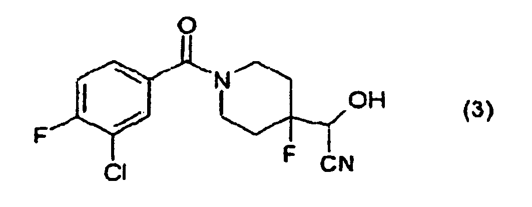 said condensation being conducted in the presence of an agent able to trap the water released during the reaction.

2. Method as in claim 1, **characterized in that** said agent capable of trapping water is a molecular sieve.

3. Method as in any of claims 1 and 2, **characterized in that** 5-methyl-pyrimidin-2-methylamine of formula (1) is obtained from a pyrimidine derivative of formula (4) 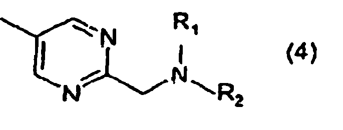 in which the radicals R₁ and R₂ independently represent a hydrogen atom and a *tert*-butyloxylcarbonyl group or a benzyloxycarbonyl group; in which R₁ and R₂ with their carrier nitrogen atom together form a phtalimido group.

4. Method as in claim 3, **characterized in that** the pyrimidine derivative of formula (4) is chosen from among the following compounds (4a), (4b) and (4c)

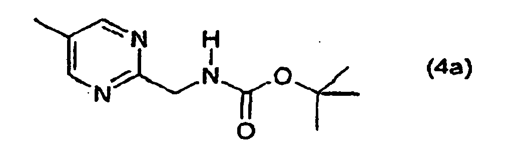

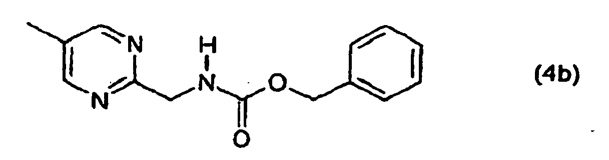

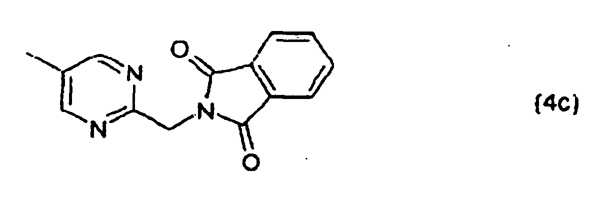

5. Method as in claim 3, **characterized in that** it uses the condensation of a glycine-derived amidine of formula (B), with a suitable dipolarophil-1,3 of formula (A) in which R is an ethoxy group or an amino or dimethylamino group. 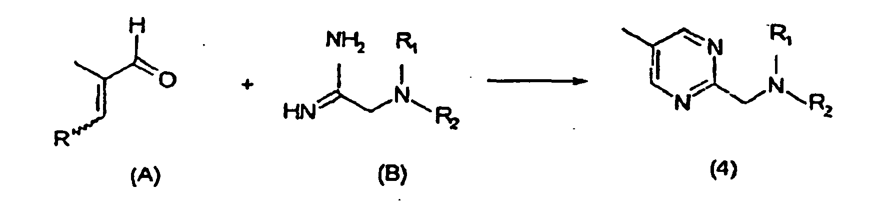 to give the compound of formula (4) in which the radicals R₁ and R₂ have the same meaning as previously.

6. Method as in claim 3, **characterized in that** it uses the condensation of a glycine-derived amidine of formula (B) with a dipolarophil-1,3 of formula (A1) in which R₃ represents a releasable group such as a C₁ to C₄ alcoxy group for example, methoxy in particular, the double bond in compound (A1) possibly being of E or Z stereochemistry indifferently. 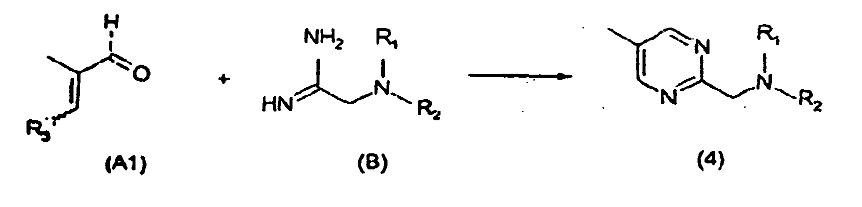 to give the compound of formula (4) in which the radicals R₁ and R₂ have the same meaning as previously.

7. Method as in any of claims 1 and 2, **characterized in that** the 5-methyl-pyrimidin-2-methylamine of formula (1) is obtained from a pyrimidine derivative of formula (4-1) 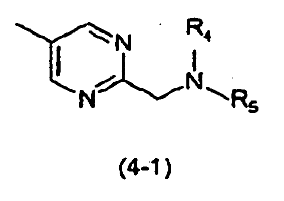 itself obtained by using the condensation of a dipolarophil-1,3 of formula (A) or of formula (A1), (A) and (A1) having the same meanings as those given for claims 5 and 6, with a glycerine-derived amidine of formula (B1) in which the radicals R₄ and R₅ together or separately represent a hydrogen atom or a protective group, in particular an alkylcarbonyl group such as a trifluorocarbonyl group

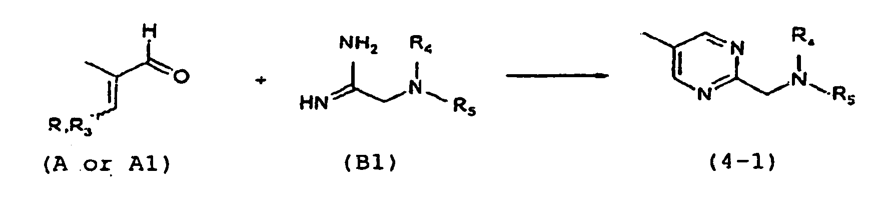

8. 5-methyl-pyrimidin-2-methylamine of formula (1)

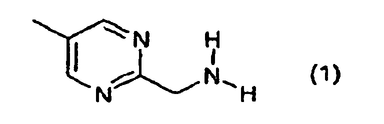

9. The pyrimidine derivatives of formula (4) 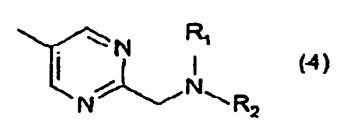 in which the radicals R₁ and R₂ separately represent a hydrogen atom and a *tert*-butyloxycarbonyl group or a benzyloxycarbonyl group; in which R₁ and R₂ with their carrier nitrogen atom together form a phtalimido group.

10. The pyrimidine derivatives according to claim 9, **characterized by** formulas (4a), (4b) and (4c)

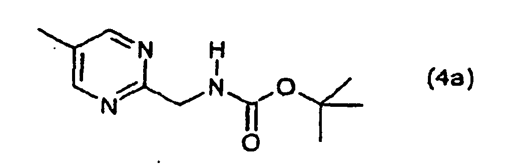

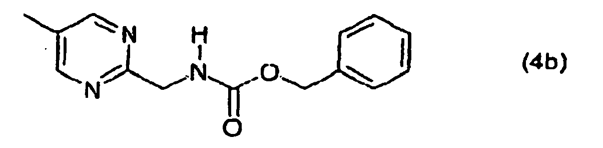

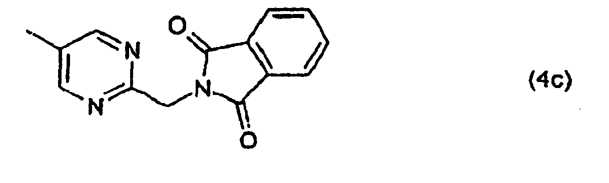

## Patentansprüche

1. Verfahren zur Herstellung von (3-Chlor-4-fluorphenyl)-(4-fluor-4-{[(5-methylpyrimidin-2-ylmethyl)-amino)-methyl}-piperidin-1-yl)-methanon der Formel (2) 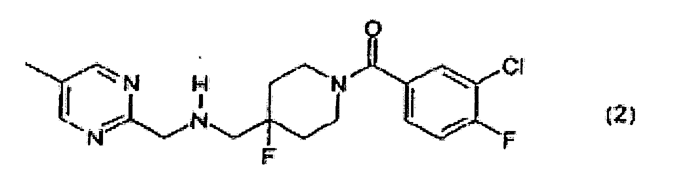 durch Kondensation von 5-Methylpyrimidin-2-methylamin der Formel (1) 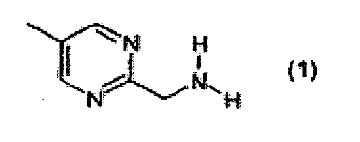 und Cyanhydrin der Formel (3) 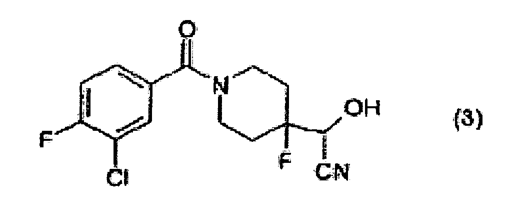 wobei die Kondensation in Gegenwart eines Agens durchgeführt wird, das imstande ist, das Wasser zurückzuhalten, das während der Reaktion freigesetzt wird.

2. Verfahren nach Anspruch 1, **dadurch gekennzeichnet, dass** es sich bei dem Agens, das zum Zurückhalten von Wasser imstande ist, um ein Mokelularsieb handelt.

3. Verfahren nach einem der Ansprüche 1 und 2, **dadurch gekennzeichnet, dass** 5-Methylpyrimidin-2-methylamin der Formel (1) aus einem Pyrimidinderivat der Formel (4) 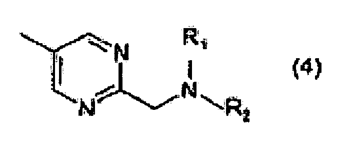 erhalten wird, wobei die Radikale R₁ und R₂ unabhängig voneinander ein Wasserstoffatom und eine tert-Butyloxylcarbonylgruppe oder eine Benzyloxycarbonylgruppe darstellen; wobei R₁ und R₂ mit ihrem Träger-Stickstoffatom zusammen eine Phthalimidgruppe bilden.

4. Verfahren nach Anspruch 3, **dadurch gekennzeichnet, dass** das Pyrimidinderivat der Formel (4) unter den folgenden Verbindungen (4a), (4b) und (4c) ausgewählt ist:

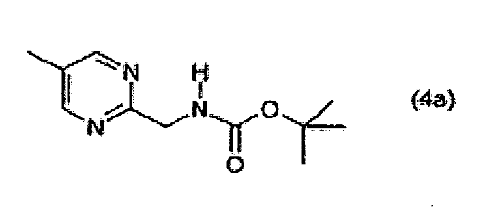

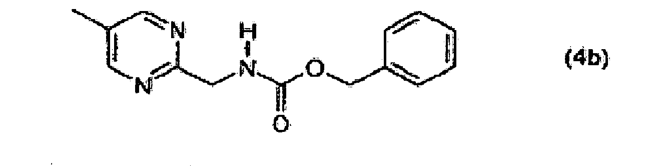

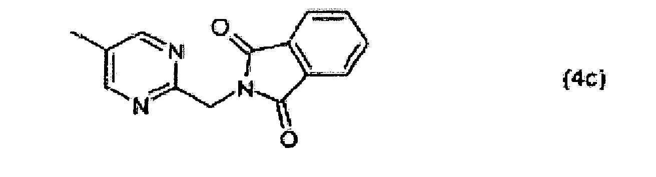

5. Verfahren nach Anspruch 3, **dadurch gekennzeichnet, dass** es die Kondensation eines von Glycin abgeleiteten Amidins der Formel (B) mit einem geeigneten Dipolarophil-1,3 der Formel (A) verwendet, wobei R für eine Ethoxygruppe oder eine Amino- oder Dimethylaminogruppe steht, 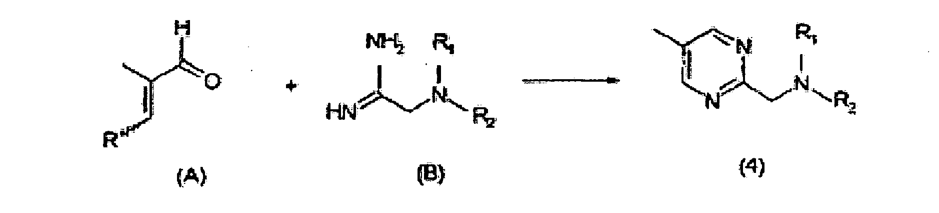 um die Verbindung der Formel (4) zu ergeben, wobei die Radikale R₁ und R₂ dieselbe Bedeutung wie zuvor haben.

6. Verfahren nach Anspruch 3, **dadurch gekennzeichnet, dass** es die Kondensation eines von Glycin abgeleiteten Amidins der Formel (B) mit einem Dipolarophil-1,3 der Formel (A1) verwendet, wobei R eine freisetzbare Gruppe wie beispielsweise C₁- oder C₄-Alcoxygruppe, insbesondere Methoxy, darstellt, und die Doppelbindung in der Verbindung (A1) möglicherweise indifferent von E- oder Z-Stereochemie ist, 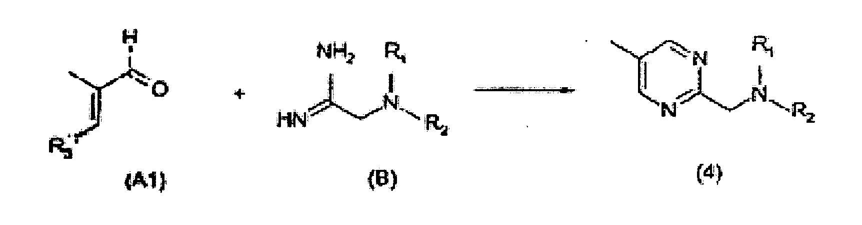 um die Verbindung der Formel (4) zu ergeben, wobei die Radikale R₁ und R₂ dieselbe Bedeutung wie zuvor haben.

7. Verfahren nach einem der Ansprüche 1 und 2, **dadurch gekennzeichnet, dass** 5-Methylpyrimidin-2-methylamin der Formel (1) aus einem Pyrimidinderivat der Formel (4-1) 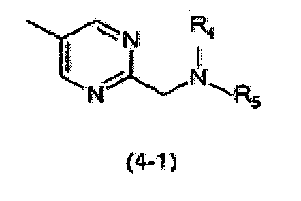 erhalten wird, das selbst durch Verwenden der Kondensation eines Dipolarophil-1,3 der Formel (A) oder der Formel (A1), wobei (A) und (A1) dieselbe Bedeutung wie jene in Anspruch 5 und 6 haben, mit einem von Glycin abgeleiteten Amin der Formel (B1) erhalten wird, wobei die Radikale R₄ und R₅ zusammen oder getrennt ein Wasserstoffatom oder eine Schutzgruppe darstellen, insbesondere eine Alkylcarbonylgruppe wie beispielsweise eine Trifluorcarbonylgruppe

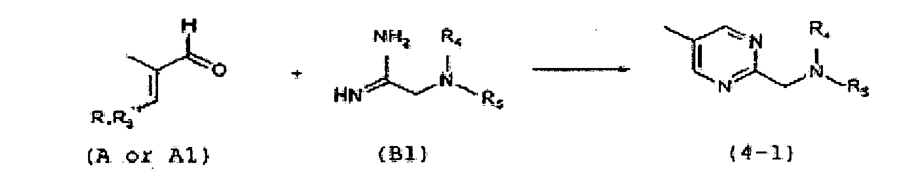

8. 5-Methylpyrimidin-2-methylamin der Formel (1)

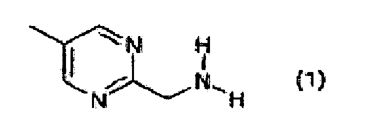

9. Pyrimidinderivate der Formel (4) 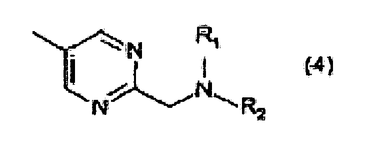 wobei die Radikale R₁ und R₂ getrennt ein Wasserstoffatom und eine tert-Butyloxylcarbonylgruppe oder eine Benzyloxycarbonylgruppe darstellen; wobei R₁ und R₂ mit ihrem Träger-Stickstoffatom zusammen eine Phthalimidgruppe bilden.

10. Pyrimidinderivate nach Anspruch 9, **gekennzeichnet durch** die Formeln (4a), (4b) und (4c)

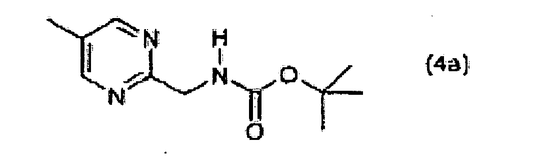

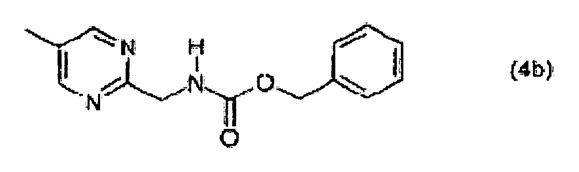

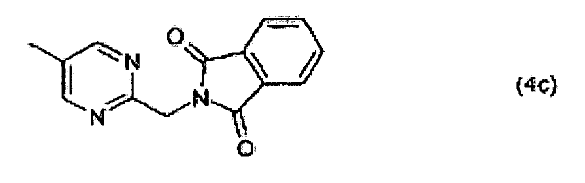

## Revendications

1. Procédé de préparation de la (3-chloro-4-fluoro-phényl)-(4-fluoro-4-{[(5-méthyl-pyrimidin-2-ylméthyl)-amino]-méthyl-}-pipéridin-1-yl)-méthanone de formule (2) 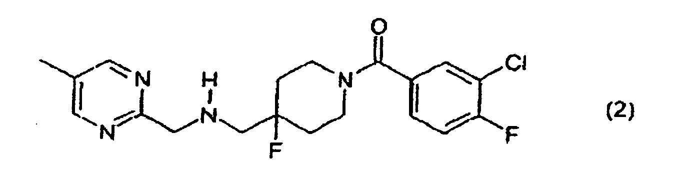 par condensation entre la 5-méthyl-pyrimidin-2-méthylamine de formule (1) 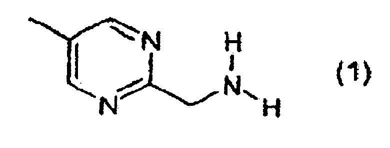 et la cyanohydridine de formule (3) 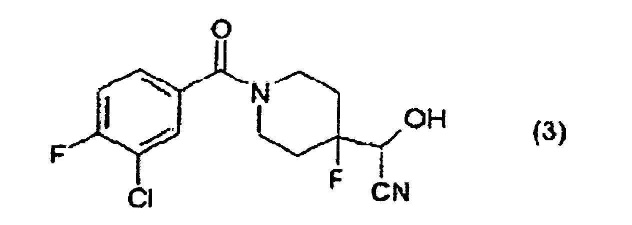 ladite condensation étant conduite en présence d'un agent capable de piéger l'eau libérée pendant la réaction.

2. Procédé selon la revendication 1, **caractérisé en ce que** ledit agent capable de piéger l'eau est un tamis moléculaire.

3. Procédé selon l'une quelconque des revendications 1 ou 2, **caractérisé en ce que** la 5-méthyl-pyrimidin-2-méthylamine de formule (1) est obtenue à partir d'un dérivé de pyrimidine de formule (4) 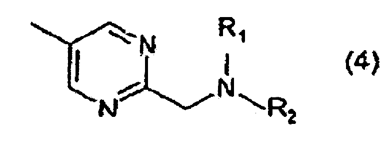 dans laquelle les radicaux R₁ et R₂ représentent indépendamment un atome d'hydrogène et un groupe *tert-*butyloxycarbonyle ou un groupe benzyloxycarbonyle ; dans laquelle R₁ et R₂ avec leur atome d'azote porteur forment ensemble un groupe phtalimido.

4. Procédé selon la revendication 3, **caractérisé en ce que** le dérivé de pyrimidine de formule (4) est choisi parmi les composés (4a), (4b) et (4c) suivants :

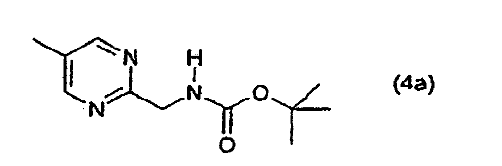

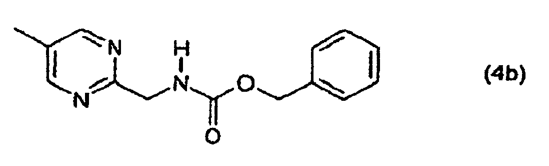

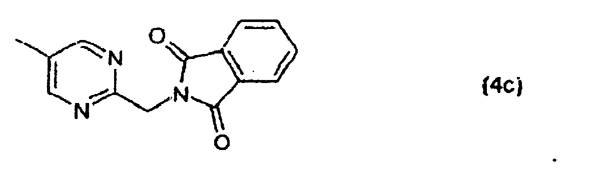

5. Procédé selon la revendication 3, **caractérisé en ce qu'**il utilise la condensation d'une amidine dérivée de la glycine de formule (B), avec un dipolarophil-1,3 approprié de formule (A) dans laquelle R est un groupe éthoxy ou un groupe amino ou diméthylamino, 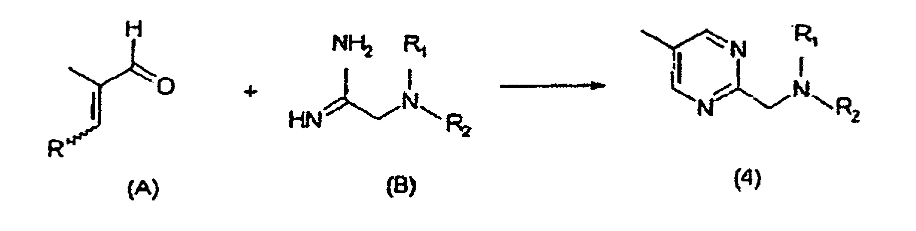 pour donner le composé de formule (4) dans laquelle les radicaux R₁ et R₂ ont la même signification que précédemment.

6. Procédé selon la revendication 3, **caractérisé en ce qu'**il utilise la condensation d'une amidine dérivée de la glycine de formule (B), avec un dipolarophil-1,3 de formule (A1) dans laquelle R₃ représente un groupe libérable tel qu'un groupe alcoxy en C₁ à C₄ par exemple, méthoxy en particulier, la double liaison dans le composé (A1) étant éventuellement de stéréochimie indifféremment E ou Z, 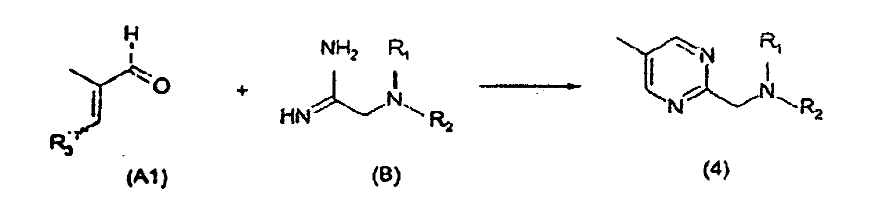 pour donner le composé de formule (4) dans laquelle les radicaux R₁ et R₂ ont la même signification que précédemment.

7. Procédé selon l'une quelconque des revendications 1 et 2, **caractérisé en ce que** la 5-méthyl-pyrimidin-2-méthylamine de formule (1) est obtenue à partir d'un dérivé de pyrimidine de formule (4-1) 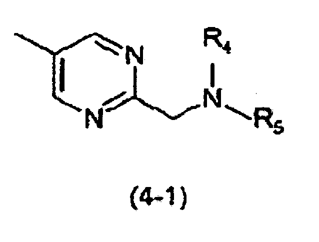 lui-même obtenu en utilisant la condensation d'un dipolarophil-1,3 de formule (A) ou de formule (A1), (A) et (A1) ayant les mêmes significations que celles données pour les revendications 5 et 6, avec une amidine dérivée de la glycérine de formule (B1) dans laquelle les radicaux R₄ et R₅ représentent ensemble ou séparément un atome d'hydrogène ou un groupe protecteur, par exemple un groupe alkylcarbonyle tel qu'un groupe trifluorocarbonyle

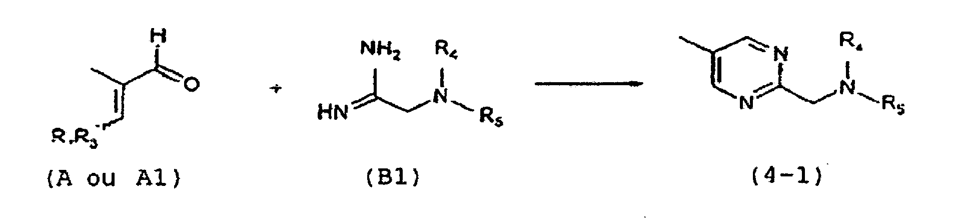

8. 5-méthyl-pyrimidin-2-méthylamine de formule (1)

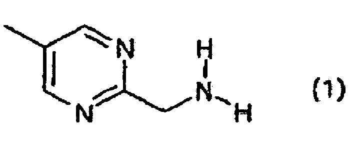

9. Dérivés de pyrimidine de formule (4) 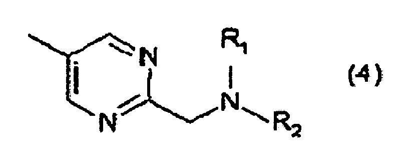 dans laquelle les radicaux R₁ et R₂ représentent séparément un atome d'hydrogène et un groupe *tert-*butyloxycarbonyle ou un groupe benzyloxycarbonyle ; dans laquelle R₁ et R₂ avec leur atome d'azote porteur forment ensemble un groupe phtalimido.

10. Dérivés de pyrimidine selon la revendication 9, **caractérisés par** les formules (4a), (4b) et (4c)
